Europäisches Patentamt

European Patent Office

Office européen des brevets

(11) Publication number: **0 095 872**
**A1**

## EUROPEAN PATENT APPLICATION

(21) Application number: 83302900.2

(22) Date of filing: 20.05.83

(51) Int. Cl.³: **A 61 F 1/04, A 61 F 1/08**

(30) Priority: 21.05.82 GB 8214835

(43) Date of publication of application: 07.12.83
Bulletin 83/49

(84) Designated Contracting States: **AT BE CH DE FR IT LI
NL SE**

(71) Applicant: **ROBERT KELLIE & SON LIMITED, Rutherford
Road Dryburgh Industrial Estate, Dundee DD2 3XF
Scotland (GB)**
Applicant: **J.E. HANGER & COMPANY LIMITED,
Roehampton Lane, Roehampton London SW15 5PL (GB)**

(72) Inventor: **Bell, Gordon Andrew, 3 Ballindean Crescent,
Dundee Scotland (GB)**
Inventor: **May, Denis Ronald William, Baronsmead
Waynefleet Tower Avenue, Esher Surrey (GB)**

(74) Representative: **Cole, Paul Gilbert et al, Hughes Clark
Andrews & Byrne 63 Lincoln's Inn Fields, London
WC2A 3JU (GB)**

(54) Improvements in or relating to knee joints for artificial legs.

(57) A knee joint for an endoskeletal artificial limb comprises a shin member 10 having a socket 11 for attachment to a shin tube 12 and a hinged portion defined by an anterior wall 14 and a medial and lateral wall 15, 16 and 1 knee member 25 having a means 26, 27 for forming thigh connection and a hinged portion that fits within the hinged portion of the shin member and is defined by an anterior wall 28 and medial and lateral walls 29, 30. A pivot pin 31 connects the knee member 25 to the shin member 10. A resiliently biased locking tongue 36 between the medial and lateral walls 29, 30 of the knee member projects through an aperture in the anterior wall 28 and presents upper and lower faces 41, 40 directed generally at right angles obliquely of the anterior wall 28 of the shin member. As the knee is unflexed upward thrust on the lower face 40 retracts the tongue 36 until the unflexed position is reached when the upper face 41 latches behind the anterior wall 14 of the shin member. A release cable 42 passes through the knee member 25 at a posterior location and is connected to the tongue 36 so tension thereon unlatches the upper face 41 from behind the anterior wall 14 to permit flexion.

EP 0 095 872 A1

# IMPROVEMENTS IN OR RELATING TO KNEE JOINTS FOR ARTIFICIAL LEGS

The present invention relates to a knee joint for an artificial leg.

In one aspect the invention provides a knee joint for an endoskeletal artificial limb comprising a shin member having a socket for attachment to a shin tube and a hinged portion defined by an anterior wall and a medial and lateral walls, a knee member having a means for forming a thigh connection and a hinged portion that fits within the hinged portion of the shin member and is defined by an anterior wall and medial and lateral walls, a pivot pin connecting the knee member to the shin member and a resiliently biased locking tongue between the medial and lateral walls of the knee member projecting through an aperture in the anterior wall and presenting upper and lower faces directed generally at right angles obliquely of the anterior wall of the shin member so that as the knee is unflexed thrust on the lower face retracts the tongue until the unflexed position is reached when the upper face latches behind the anterior wall, and a release cable that passes through the knee member at a posterior location and is connected to the tongue so tension thereon unlatches the upper face from behind the anterior wall to permit flexion.

The aforesaid knee joint is simple, robust and presents a generally smooth and streamlined external appearance. It has the advantage that the release cable which may be a Bowden cable or the like, is wholly within the knee member and is not subjected to bending when the knee is flexed. And its posterior exit point means that it can be curved around and fitted to either a left handed or to a right handed socket.

If desired swing phase control means may be connected at its upper end to the knee unit at a pivot point posterior of the knee pivot and swingably or pivotally located to the shin unit at a position at or adjacent to the shin tube, substantially the whole of the swing phase control means being below the knee pivot.

Preferably the unflexed position of the knee is defined by abutment between the upper face of the tongue and an adjustable stop screw that passes through the anterior wall of the shin member. Apertures in the medial and lateral walls of the knee member preferably carry outer races of respective ball bearings whose inner races fit onto the pivot pin, a spacer tube fitting over the pivot pin between the inner races, and the end extremities of the inner ball races locating against the medial and lateral walls of the knee member so as to reduce play in the joint. The pivot pin may be constituted by a hollow tubular centre section threaded internally at its ends and by a pair of bolts that pass through the medial and lateral walls of the shin member and threadedly engage the centre section.

An embodiment of the invention will now be described with reference to the accompanying drawings, in which:

Figure 1 is a sectional side view of a knee joint according to the invention in a partially flexed position;

Figure 2 is a rear view of the joint; and

Figure 3 is a front view of the joint.

In the drawings a shin unit 10 is a light alloy casting formed to define a socket 11 that fits onto shin tube 12 to which it is held by clamping screws 13 and a hinge socket defined by an anterior wall 14 and by medial and lateral walls 15, 16. An extension bumper 17 is fixed (eg. by screws) to the top face of anterior wall 14 and is formed in a rubbery material to take up impact as the joint reaches the straight position. Fitted into a threaded hole through wall 14 is an adjustable stop screw 19 which may be clamped in position by locking screw 20 both of which can be adjusted from in front of the knee. The purpose of stop screw 19 is to permit adjustment of the unflexed position of the leg.

The knee unit 25 comprises a socket which is also a light alloy casting formed with a part spherical seat 26 for a coupling member (European Patent Application No.81305762.7) and provided with three adjustable clamping screws 27 or a four-screw socket of the kind described in UK Patent Specifications No.1307919 or No. 1494706 or another form of thigh connection, for example a flat plate bolted to the knee unit. Beneath the socket is a hinged member that fits within the hinge member of shin unit 10 and is defined by a part cylindrical anterior wall 28 and medial and lateral walls 29 and 30. Ears 34 on the knee unit contact walls 15, 16 of the shin unit at approximately 130° of knee flexion and stop over-flexion of the knee. A knee bolt 31 passes through walls 15, 30, 29, 16 and through a pair of high load capacity ball bearings 32 that fit into the medial and lateral walls 29, 30 of knee unit 25 and are kept apart by spacer tube 33. Outer races of the ball bearings fit tightly into apertures in the medial and lateral walls 29, 30 and the extremities 32a of the inner races that fit over the bolt 31 extend outwardly so that their ends fit tightly between walls 15, 16 of the shin member to minimise play in the joint. The knee bolt is formed in three pieces, a hollow central tube internally threaded at its ends and a pair of bolts that fit into countersunk holes in the lateral walls of the shin member and engage opposed ends of the central tube. Above the anterior wall is formed an abutment 35 that projects forwardly and when the joint is in extension contacts bumper 17 to prevent over-extension of the joint. Also formed in the anterior wall is a cut-out through which protrudes a locking tongue 36 pivoted adjacent its lower edge at 37 to the shin member and biased by spring 38 so that its top edge locates against the top of the cut-out at 39. Front faces 40, 41 of the tongue 36 are directed approximately at right angles to one another the face 41 having a slight convex curvature as shown, though it could also be planar like face 40. A release cable 42 passes through a hole 43 through the posterior portion of the socket and through the tongue 36 adjacent its top edge, terminating in stud 44. Sheath 45 of cable 42 is jammed into hole 43.

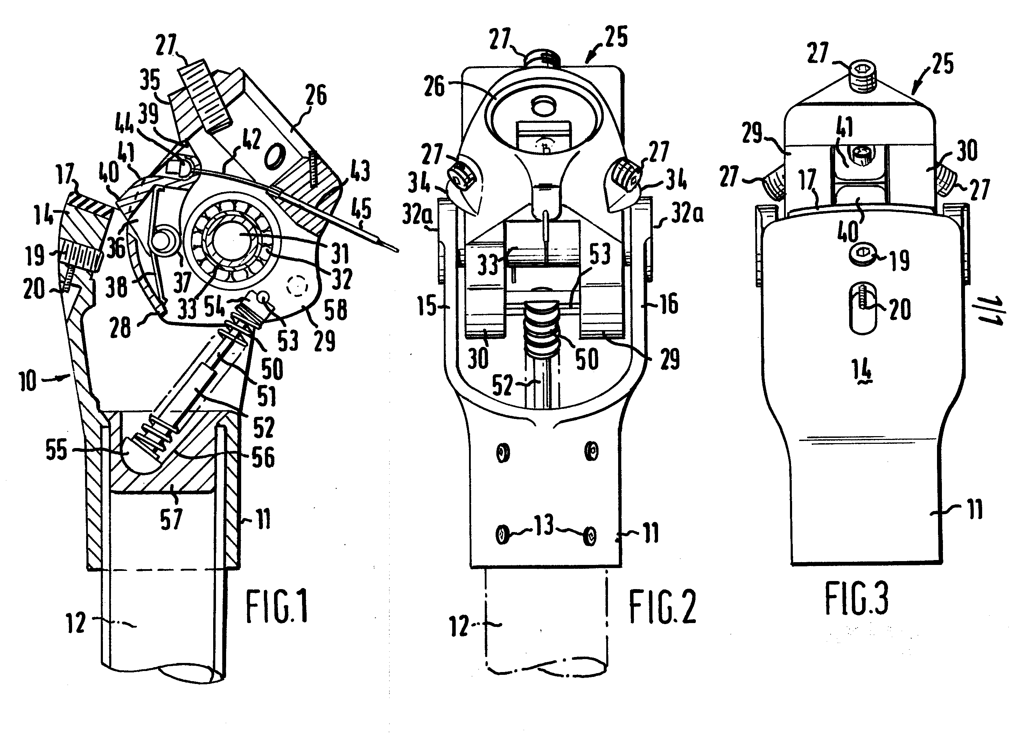

As the knee is straightened from the position shown in Figure 1, surface 40 of the tongue 36 slides over bumper 17 and causes clockwise rotation of the tongue 36 towards a retracted position which is retained until the joint reaches its unflexed position when the tongue snaps into its latching engagement with the flat or convex surface 41 engaged behind screw 19. For release of the knee, cable 42 is pulled which rotates pawl 36 clockwise and unlatches face 41 from adjustable lock screw 19. It will be noted that substantially no bending of the release cable occurs during flexion and extension of the knee.

A coil spring 50 may be fitted between the knee and shin units and is prevented from bending by pin 51 and tube 52. The top of pin 51 is formed with an enlarged end 54 that provides a thrust surface for spring 50 and is

5 0095872

connected to a pin 53 that is pivotally mounted in medial and lateral walls 29, 30. The base of tube 52 is formed with an enlarged end 55 that provides a thrust surface for spring 50 and has a part spherical lower surface that as the knee flexes and straightens pivots on a conforming surface 56 of an end fitting 57 that is a push fit into the top end of shin tube 12. Thus for the more active patient the locking tongue or pawl 16 may be kept disengaged so that the knee can flex during walking and the spring 50 provides a degree of control and resistance to flexion.

In a yet further version (not illustrated) for even more active patients the shin unit 10 is extended downwardly to accomodate a swing phase control or damper unit that is mounted between pivot point 58 on the knee unit and a pivot point on the downwardly extended shin unit.

CLAIMS:

1. A knee joint for an endoskeletal artificial limb comprising a shin member 10 having a socket 11 for attachment to a shin tube 12 and a hinged portion defined by an anterior wall 14 and a medial and lateral wall 15, 16, a knee member 25 having a means 26, 27 for forming thigh connection and a hinged portion that fits within the hinged portion of the shin member and is defined by an anterior wall 28 and medial and lateral walls 29, 30, a pivot pin 31 connecting the knee member 25 to the shin member 10, and a resiliently biased locking tongue 36 between the medial and lateral walls 29, 30 of the knee member projecting through an aperture in the anterior wall 28 and presenting upper and lower faces 41, 40 directed generally at right angles obliquely of the anterior wall 28 of the shin member so that as the knee is unflexed upward thrust on the lower face 40 retracts the tongue 36 until the unflexed position is reached when the upper face 41 latches behind the anterior wall 14 of the shin member, and a release cable 42 that passes through the knee member 25 at a posterior location and is connected to the tongue 36 so tension thereon unlatches the upper face 41 from behind the anterior wall 14 to permit flexion.

2. A knee joint according to claim 1, wherein the unflexed position of the knee is defined by abutment between the upper face 41 of the tongue 36 and an adjustable stop screw 19 that passes through the anterior wall 14 of the shin member 10.

3. A knee joint according to claim 1 or 2, wherein apertures in the lateral walls of the knee member carry outer races of respective ball bearings 32 whose inner races 32a fit onto the pivot pin 31, a spacer tube 33 fitting over the pivot pin 31 between the inner races 32a, and the end extremities of the inner ball races 32a locating against the medial and lateral wall 29, 30 of the knee member so as to reduce play in the joint.

4. A knee joint according to claim 1, 2 or 3, wherein

0095872

the pivot pin is constituted by a hollow tubular centre
section threaded internally at its ends and by a pair of
bolts that pass through the medial and lateral wall 15,16
of the shin member 10 and threadedly engage the centre
section.

5.    A knee joint according to any preceding claim,
wherein portions of the knee member 10 define an abutment
surface 35 projecting forwardly above the anterior wall 28
that in the unflexed position of the knee locate against a
resilient buffer member 17 attached to the top of the
anterior wall 14 of the shin member 10.

6.    A knee joint according to any preceding claim wherein
a two component extensible  pin  51, 52 is pivoted  to the
knee member posterior of the knee pivot and rotatably
engages a socket 56 in the shin member, and a coil spring
50 biases the components of the pin apart thereby
unflexing the leg.

7.    A knee joint according to any preceding claim,
wherein the knee joint and the shin joint are castings in
light alloy.

8     A knee joint for an endoskeletal limb substantially
as hereinbefore described with reference to and as
illustrated in the accompanying drawings.

FIG.1

FIG.2

FIG.3

1/1

0095872

0095872

European Patent
Office

**EUROPEAN SEARCH REPORT**

Application number

| | DOCUMENTS CONSIDERED TO BE RELEVANT | | EP 83302900.2 |
|---|---|---|---|

| Category | Citation of document with indication, where appropriate, of relevant passages | Relevant to claim | CLASSIFICATION OF THE APPLICATION (Int. Cl. 3) |
|---|---|---|---|
| A | US - A - 3 551 915 (C. WOODALL) <br> * Fig. 1,2,5 * | 1,3,6 | A 61 F  1/04 <br> A 61 F  1/08 |
| A | US - A - 2 277 548 (M.S. JACKSON) <br> * Totality * | 1,3,4 | |
| A | US - A - 1 213 114 (L.C.LAWRENCE) <br> * Fig. 2; page 2, lines 28-32 * | 1,6 | |
| A | US - A - 2 249 365 (L.C. SANSBURY) <br> * Fig. 3 * | 1,5 | |
| A | GB - A - 712 942 (W.A.D. BLATCH-FORD) <br> * Totality * | 1 | |
| P | EP - A2 - 0 054 391 (R. KELLIE & SON LTD.) <br> * Fig. 1; page 6, line 2 - page 7, line 7 * <br> & GB-A-2 089 216 (23-06-1983) <br> & DK-A- 5 403/81 (09-06-1983) | 1-6 | |

**TECHNICAL FIELDS SEARCHED (Int. Cl. 3)**

A 61 F  1/00

The present search report has been drawn up for all claims

| Place of search | Date of completion of the search | Examiner |
|---|---|---|
| VIENNA | 15-09-1983 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPC Form 1503 03 82